# EUROPÄISCHE PATENTSCHRIFT

(11) **EP 3 370 685 B1**
(45) Veröffentlichungstag und Bekanntmachung des Hinweises auf die Patenterteilung: **26.08.2020**
(21) Anmeldenummer: 16782061.2
(22) Anmeldetag: 17.10.2016
(51) Int. Cl.: A61K 8/34, A61Q 19/00, A61K 8/04, A61K 8/06

(54) **KOSMETISCHER SCHAUM AUS EINER EMULSION ENTHALTEND GLYCERIN UND ALKOHOL**
COSMETIC FOAM FROM AN EMULSION CONTAINING GLYCERIN AND ALCOHOL
MOUSSE COSMÉTIQUE À PARTIR D'UNE ÉMULSION CONTENANT DE LA GLYCÉRINE ET DE L'ALCOOL

(30) Priorität: 04.11.2015 DE 102015221568
(43) Veröffentlichungstag der Anmeldung: 12.09.2018
(73) Patentinhaber: Beiersdorf AG, 20253 Hamburg (DE)
(72) Erfinder: SKUBSCH, Kerstin, 25497 Prisdorf (DE); MÜLLER, Claudia, 25499 Tangstedt (DE); RUPP, Katrin, 22417 Hamburg (DE); ECKERT, Julia, 22303 Hamburg (DE); SCHULZ, Sabine, 22159 Hamburg (DE)
(86) Internationale Anmeldenummer: PCT/EP2016/074834
(87) Internationale Veröffentlichungsnummer: WO 2017/076616

(56) Entgegenhaltungen:
- EP-A1- 2 335 675
- WO-A1-2012/146576
- US-A- 5 866 040
- US-A1- 2009 098 074
- Basf: "Emulsifiers & Cream Bases - Specialties Multi-skilled actors in your formulation Product Information Application & Performance Application Texture Benefits Trade Name INCI Name Emulsion Type HLB* Eco- status EO Face/Body Care Sun Care Color Care AP/ Deo Spray Lotion Creamy texture SELLER MAKES NO WA", , 12 August 2019 (2019-08-12), XP055612327, Retrieved from the Internet: URL:https://ami-ingredients.fr/wp-content/ uploads/2018/06/Emulsifiants-et-bases-auto -emulsionnantes.pdf [retrieved on 2019-08-12]
- DATABASE GNPD [Online] MINTEL; 29 June 2014 (2014-06-29), anonymous: "Cooling Mousse SPF 50+ PA+++", XP055612410, retrieved from www.gnpd.com Database accession no. 2476069
- DATABASE GNPD [Online] MINTEL; 12 March 2008 (2008-03-12), anonymous: "Active Hydration Mousse", XP055612411, retrieved from www.gnpd.com Database accession no. 874606

## Beschreibung

Die vorliegende Erfindung betrifft einen kosmetischen Schaum aus einer O/W-Emulsion enthaltend Glycerin sowie ein- und/oder mehrwertige Alkohole mit einer Kohlenstoff-kettenlänge von weniger als 12 C-Atomen und einem die Emulsion aufschäumenden Gas oder Gasgemisch aus Propan, n-Butan und/oder Isobutan, wobei die Emulsion frei ist von Polyethylenglycol-Derivaten (PEG-Derivaten). Die Erfindung betrifft ferner eine Aerosoldose mit Entnahmeventil enthaltend einen derartigen Schaum.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren immer das Ziel der Menschen gewesen. Einen wesentlichen Anteil an einem schönen und attraktiven Äußeren hat dabei der Zustand und das Aussehen der Haut.

Damit die Haut ihre biologischen Funktionen im vollen Umfang erfüllen kann, bedarf sie der regelmäßigen Reinigung und Pflege. Die Reinigung der Haut dient dabei der Entfernung von Schmutz, Schweiß und Resten abgestorbener Hautpartikel, die einen idealen Nährboden für Krankheitserreger und Parasiten aller Art bilden. Hautpflegeprodukte dienen meist der Befeuchtung und Rückfettung der Haut. Häufig sind ihnen Wirkstoffe zugesetzt, welche die Haut regenerieren und beispielsweise ihre vorzeitige Alterung (z.B. das Entstehen von Fältchen, Falten) verhindern und vermindern sollen.

Hautpflegeprodukte bestehen (ebenso wie einige Hautreinigungsprodukte) in der Regel aus Emulsionen. Unter Emulsionen versteht man im Allgemeinen heterogene Systeme, die aus zwei nicht oder nur begrenzt miteinander mischbaren Flüssigkeiten bestehen, die üblicherweise als Phasen bezeichnet werden und bei denen eine der beiden Flüssigkeiten in Form feinster Tröpfchen in der anderen Flüssigkeit dispergiert ist. Äußerlich und mit bloßem Auge betrachtet erscheinen Emulsionen homogen.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-ÖI-Emulsion (W/O-Emulsion, z. B. Butter) handelt es sich um das umgekehrte Prinzip, wobei der Grundcharakter hier durch das Öl bestimmt wird.

Neben der "klassischen" Anwendung, Emulsionen direkt aus dem Vorratsbehältnis auf die Haut aufzutragen, gibt es auch eine geringere Anzahl von Anwendungen, bei denen die Emulsion zur Anwendung auf der Haut mit Hilfe eines Treibgases aufgeschäumt wird. Aufgeschäumte Emulsionen weisen aufgrund ihrer Schaumkonsistenz ein besonderes Hautgefühl auf, dass von den Anwendern als besser auf der Haut verteilbar wahrgenommen wird mit einem leichteren und weniger klebrigen Hautgefühl. Die Anwendung der moussigen Textur macht den Anwendern außerdem Spaß.

Nachteilig am Stande der Technik ist jedoch der Umstand, dass man für die Herstellung kosmetischer Emulsionsschäume den Zubereitungen Polyethylenglycole und/oder Polyethylenglycol-Derivate (PEG-Derivate) zusetzen muss. PEG-Derivate sind Verbindungen mit Alkohol- oder Säurefunktion, die mit Polyethylenglycolen ganz oder teilweise verethert oder verestert sind, um den Schaum über einen längeren Zeitraum stabil zu halten. Diese PEG-Derivate sind beim Verbraucher jedoch zunehmend unerwünscht, da ihnen von einigen Wissenschaftlern die gesundheitliche Unbedenklichkeit abgesprochen wird. Ob diese Bedenken wissenschaftlich begründet sind, kann im Rahmen der vorliegenden Erfindung dahingestellt bleiben. Tatsache ist hingegen, dass der Verbraucher zunehmend nach Kosmetika verlangt, die "PEG frei" sind.

Es war daher die Aufgabe der vorliegenden Erfindung, einen sensorisch attraktiven, über einen längeren Zeitraum stabilen kosmetischen Schaum auf der Basis einer aufgeschäumten Emulsion zu entwickeln, die "PEG frei" ist.

Kosmetische O/W-Emulsionen haben darüber hinaus den Nachteil, dass sie besonders stark konserviert werden müssen, um über einen längeren Zeitraum mikrobiologisch einwandfrei zu sein. Hierzu werden insbesondere Konservierungsmittel aus der Gruppe der Parabene verwendet. Konservierungsmittel und insbesondere Parabene stehen (ob zu Recht oder zu Unrecht kann im Rahmen dieser Offenbarung dahingestellt bleiben) bei den Verbrauchern in dem Ruf, hinsichtlich ihrer Gesundheitsverträglichkeit und Umweltverträglichkeit nicht ganz unbedenklich zu sein.

Es war daher die Aufgabe der vorliegenden Erfindung, einen mikrobiologisch stabilen kosmetischen Schaum auf der Basis einer O/W-Emulsion zu entwickeln, der "parabenfrei" ist.

Darüber hinaus war dem Fachmann an sich bekannt, dass Alkohole zur Konservierung kosmetischer O/W-Emulsionen verwendet werden können, beispielsweise Ethanol oder Alkandiole wie 1,2-Oktandiol (Caprylylglycol). Von Alkoholen ist jedoch bekannt, dass sie den Schaum einer aufgeschäumten Emulsion destabilisieren, so dass dieser schnell in sich zusammenfällt. Darüber hinaus sorgt insbesondere Ethanol dafür, dass die Haut gereizt wird und zur Austrocknung neigt.

Es war daher die Aufgabe der vorliegenden Erfindung, einen mikrobiologisch stabilen, "parabenfreien" Schaum auf der Basis einer O/W-Emulsion zu entwickeln, der ein allenfalls geringes Hautreizungspotential aufweist und zu einem stabilen Schaum führt.

Nicht zuletzt sind mit Propan, n-Butan ("Butan") oder Isobutan aufgeschäumte Schäume in der Regel relativ leicht brennbar. Dieses Problem wird verstärkt, wenn die Zubereitung weitere brennbare Inhaltsstoffe wie Ethanol enthält. Für derartige Systeme müssen nach dem Stand der Technik bei der Herstellung, Lagerung, Aufbewahrung im Vorratsbehältnis und bei der Anwendung besondere Brandschutzmaßnahmen getroffen werden.

Es war daher die Aufgabe der vorliegenden Erfindung, eine mit Propan, n-Butan ("Butan") oder Isobutan aufgeschäumte oder aufschäumbare O/W-Emulsion zu entwickeln, bei der keine besonderen Brandschutzmaßnahmen zu beachten sind.

Überraschend gelöst wird die Aufgabe durch einen kosmetischen Schaum gemäß Anspruch 1.

Dabei zählen Glycerin, Propylenglycol und Butylenglycol erfindungsgemäß (Definition) nicht zu den ein- und/oder mehrwertigen Alkohole mit einer Kohlenstoffkettenlänge von weniger als 12 C-Atomen.

Zwar kennt der Stand der Technik die EP1277455, EP1014916, DE 10138495, WO2012/146576, US5866040, EP2335675, US2009/098074 oder die Datenbankeinträge in der GNPD Datenbank Mintel mit den Eintragungsnummern 2476069 und 874606, doch konnten diese Schriften nicht den Weg zur vorliegenden Erfindung weisen.

Es ist erfindungsgemäß von besonderem Vorteil, wenn die Emulsion frei ist von Seifen (wobei eine Emulsion erfindungsgemäß als "frei von Seifen" gilt, wenn sie weniger als 0,2 Gewichts-%, bezogen auf das Gesamtgewicht der Emulsion an Seifen enthält). Dabei werden unter "Seifen" erfindungsgemäß Natrium- oder Kaliumsalze von Fettsäuren verstanden.

Es ist erfindungsgemäß, wenn die Emulsion frei ist von Tensiden mit einem HLB-Wert von größer 12, wobei Natriumcetearylsulfat erfindungsgemäß nicht zu den Tensiden gezählt wird.

Dieser Sachverhalt ist umso überraschender, da Seifen und Tenside üblicherweise zur Stabilisierung von kosmetischen Schäumen erforderlich sind. Hingegen führt bei den erfindungsgemäßen Schäumen das Weglassen der Tenside hingegen zu einer Stabilisierung des Schaums und zu einem deutlich cremigeren Schaum, der einfacher zu verteilen ist.

In dieser erfindungsgemäß vorteilhaften Ausführungsform sind die erfindungsgemäßen Schäume überraschend stabiler und cremiger als unter Zusatz dieser oberflächenaktiven Verbindungen und verfügen über ein signifikant geringeres Hautreizungspotential.

Es ist erfindungsgemäß vorteilhaft, wenn der kosmetische dadurch gekennzeichnet ist, dass die Emulsion von 3 bis 20 Gewichts-%, bezogen auf das Gesamtgewicht der Emulsion Glycerin enthält. Erfindungsgemäß bevorzugt ist dabei ein Gehalt von 5 bis 15 Gewichts-%, bezogen auf das Gesamtgewicht der Emulsion.

Darüber hinaus ist es für den erfindungsgemäßen Schaum erfindungsgemäß vorteilhaft, wenn die Gesamtkonzentration an ein- und mehrwertigen Alkoholen mit einer Kohlenstoffkettenlänge von weniger als 12 C-Atomen in der Emulsion 0,5 bis 15 Gewichts-%, bezogen auf das Gesamtgewicht der Emulsion, beträgt. Dabei entspricht diese Gesamtkonzentration der Einzelkonzentration für den Fall, dass die Emulsion nur einen Alkohol mit einer Kohlenstoffkettenlänge von weniger als 12 C-Atomen enthält.

Erfindungsgemäß vorteilhafte Schäume sind dadurch gekennzeichnet, dass die ein- und/oder mehrwertigen Alkohole mit einer Kohlenstoffkettenlänge von weniger als 12 C-Atomen ausgewählt werden aus der Gruppe der Verbindungen Ethanol, Phenoxyethanol, 1,2-Pentandiol, 1,2-Hexandiol, 1,2-Octandiol, 2-Methyl-1,3-propandiol, 1,2-Decandiol, Ethylhexylglycerin, Benzylalkohol, Phenethylalkohol.

Enthält der erfindungsgemäße Schaum Ethanol, so ist es erfindungsgemäß bevorzugt, diesen Stoff in einer Konzentration von 0,5 bis 10 Gewichts-%, bezogen auf das Gesamtgewicht der Emulsion, einzusetzen.

Enthält der erfindungsgemäße Schaum Phenoxyethanol, so ist es erfindungsgemäß bevorzugt, diesen Stoff in einer Konzentration von 0,1 bis 1 Gewichts-%, bezogen auf das Gesamtgewicht der Emulsion, einzusetzen.

Enthält der erfindungsgemäße Schaum 1,2-Pentandiol, so ist es erfindungsgemäß bevorzugt, diesen Stoff in einer Konzentration von 0,1 bis 5 Gewichts-%, bezogen auf das Gesamtgewicht der Emulsion, einzusetzen.

Enthält der erfindungsgemäße Schaum 1,2-Hexandiol, so ist es erfindungsgemäß bevorzugt, diesen Stoff in einer Konzentration von 0,1 bis 5 Gewichts-%, bezogen auf das Gesamtgewicht der Emulsion, einzusetzen.

Enthält der erfindungsgemäße Schaum 1,2-Octandiol, so ist es erfindungsgemäß bevorzugt, diesen Stoff in einer Konzentration von 0,1 bis 5 Gewichts-%, bezogen auf das Gesamtgewicht der Emulsion, einzusetzen.

Enthält der erfindungsgemäße Schaum 2-Methyl-1,3-propandiol, so ist es erfindungsgemäß bevorzugt, diesen Stoff in einer Konzentration von 0,5 bis 10 Gewichts-%, bezogen auf das Gesamtgewicht der Emulsion, einzusetzen.

Enthält der erfindungsgemäße Schaum 1,2-Decandiol, so ist es erfindungsgemäß bevorzugt, diesen Stoff in einer Konzentration von 0,1 bis 5 Gewichts-%, bezogen auf das Gesamtgewicht der Emulsion, einzusetzen.

Enthält der erfindungsgemäße Schaum Ethylhexylglycerin, so ist es erfindungsgemäß bevorzugt, diesen Stoff in einer Konzentration von 0,1 bis 2 Gewichts-%, bezogen auf das Gesamtgewicht der Emulsion, einzusetzen.

Enthält der erfindungsgemäße Schaum Benzylalkohol, so ist es erfindungsgemäß bevorzugt, diesen Stoff in einer Konzentration von 0,1 bis 2 Gewichts-%, bezogen auf das Gesamtgewicht der Emulsion, einzusetzen.

Enthält der erfindungsgemäße Schaum Phenethylalkohol, so ist es erfindungsgemäß bevorzugt, diesen Stoff in einer Konzentration von 0,1 bis 3 Gewichts-%, bezogen auf das Gesamtgewicht der Emulsion, einzusetzen.

Erfindungsgemäß vorteilhafte Ausführungsformen der vorliegenden Erfindung sind dadurch gekennzeichnet, dass der Schaum gebildet wird aus 90-96 Gew.-% Emulsion und 4 bis 10 Gew.-% Gas bzw. Gasgemisch. Erfindungsgemäß bevorzugte Ausführungsformen sind dadurch gekennzeichnet, dass der Schaum gebildet wird aus 92-94 Gew.-% Emulsion und 6 bis 8 Gew.-% Gas bzw. Gasgemisch. Dass sich die erfindungsgemäßen Schäume bereits mit einer derartig geringen Menge an Gas gleichmäßig aufschäumen lassen, war dabei für den Fachmann nicht vorhersehbar. Die Ursache dafür ist die überraschend gute und einfache gleichmäßige Verteilung des Gases in der Emulsion.

Es ist erfindungsgemäß vorteilhaft, wenn als Gas ein Gasgemisch aus Butan, Iso-Butan und/oder Propan eingesetzt wird. Das Mischungsverhältnis der Gase variiert je nach Druckstufe z.B.:
Druckstufe 2,7 bar: 60 % Butan, 20% Propan und 20 % Iso-Butan.
Druckstufe 3,0 bar: 5,3 % Butan, 15,3% Propan und 79,4 % Iso-Butan.
Druckstufe 3,5 bar: 5 % Butan, 23% Propan und 72 % Iso-Butan.
Erfindungsgemäß bevorzugt sind die Druckstufen 2,7 bar, 3,0 bar und 3,5 bar.
Besonders bevorzugt sind die Druckstufen 3,0 bar und 3,5 bar.

Es ist erfindungsgemäß von Vorteil, wenn die Emulsion frei ist von Parabenen, Isothiazolinonen und 3-Iodpropargyl-*N*-butylcarbamat (IPBC) sowie halogenhaltigen Verbindungen.

Erfindungsgemäß vorteilhafte Ausführungsformen der vorliegenden Erfindung werden auch dadurch erhalten, dass die Emulsion EDTA und/oder Betain enthält.

Enthält die Emulsion EDTA so ist es erfindungsgemäß von Vorteil, wenn der Gehalt an EDTA von 0,01 bis 2 Gew.-%, bezogen auf das Gesamtgewicht der Emulsion beträgt.

Enthält die Emulsion Betain, so ist es erfindungsgemäß von Vorteil, wenn der Gehalt an Betain von 0,001 bis 6 Gew.-%, bezogen auf das Gesamtgewicht der Emulsion beträgt.

Erfindungsgemäß vorteilhafte Ausführungsformen der vorliegenden Erfindung sind dadurch gekennzeichnet, dass die Lipidphase der Emulsion einen oder mehrere Fettalkohole, Mandelöl, Kakaobutter und/oder Sheabutter enthält. Dabei ist es insbesondere überraschend gewesen, dass die Emulsionen, wenn sie Stearylalkohol bzw. Behenylalkohol enthalten, zu leicht und gleichmäßig mit dem Gas vermischbaren Zubereitungen führen, da beide Fettalkohole zu einer Erhöhung der Viskosität der Emulsion führen, was die Vermischbarkeit mit dem Gas(-gemisch) grundsätzlich erschweren sollte.

Die erfindungsgemäß vorteilhafte Einsatzkonzentration für Stearylalkohol beträgt von 0,01 bis 4 Gew.-%, bezogen auf das Gesamtgewicht der Emulsion.

Die erfindungsgemäß vorteilhafte Einsatzkonzentration für Behenylalkohol beträgt von 0,01 bis 4 Gew.-%, bezogen auf das Gesamtgewicht der Emulsion.

Die erfindungsgemäß vorteilhafte Einsatzkonzentration für Mandelöl beträgt von 0,01 bis 5 Gew.-%, bezogen auf das Gesamtgewicht der Emulsion.

Die erfindungsgemäß vorteilhafte Einsatzkonzentration für Kakaobutter beträgt von 0,01 bis 5 Gew.-%, bezogen auf das Gesamtgewicht der Emulsion.

Die erfindungsgemäß vorteilhafte Einsatzkonzentration für Sheabutter beträgt von 0,01 bis 5 Gew.-%, bezogen auf das Gesamtgewicht der Emulsion.

Erfindungsgemäß vorteilhafte Ausführungsformen der vorliegenden Erfindung sind dadurch gekennzeichnet, dass der Gewichtsanteil der Lipidphase an der Emulsion 7 bis 25 Gew.-%, bezogen auf das Gesamtgewicht der Emulsion, beträgt.

Es ist erfindungsgemäß von Vorteil, wenn die Emulsion weder Mineralöl noch Silikonöl enthält.

Silikonöle können jedoch auch in Mengen von kleiner 1 Gew.-%, bezogen auf das Gesamtgewicht der Emulsion enthalten sein.

Es ist vorteilhaft im Sinne der vorliegenden Erfindung, wenn der kosmetische Schaum dadurch gekennzeichnet ist, dass die Emulsion frei ist von Verdickungsmitteln wie z.B. Siliciumdioxid, Aluminiumsilikate, Polysaccharide bzw. deren Derivate, z. B. Hyaluronsäure, Xanthangummi, Hydroxypropylmethylcellulose, Polyacrylate, wie Carbopole, beispielsweise Carbomere und Acrylates/C10-30 Alkyl Acrylate Crosspolymer.

Die Wasserphase der erfindungsgemäßen Emulsion kann übliche kosmetische Hilfsstoffe enthalten.

Es ist erfindungsgemäß vorteilhaft, wenn die erfindungsgemäße Emulsion Emulgatoren in einer Gesamtkonzentration von 0,1 bis 8 Gewichts-%, bezogen auf das Gesamtgewicht der Emulsion, enthält. Dabei entspricht diese Konzentration der vorteilhaften Einzelkonzentration für den Fall, dass die Emulsion nur einen Emulgator enthält.

Enthält die erfindungsgemäße Emulsion Glycerylstearatcitrat als Emulgator, so ist es erfindungsgemäß bevorzugt, diesen Emulgator in einer Konzentration von 0,5 bis 5 Gewichts-%, bezogen auf das Gesamtgewicht der Emulsion einzusetzen.

Enthält die erfindungsgemäße Emulsion Cetylphosphat als Emulgator, so ist es erfindungsgemäß bevorzugt, diesen Emulgator in einer Konzentration von 0,05 bis 2 Gewichts-%, bezogen auf das Gesamtgewicht der Emulsion einzusetzen.

Enthält die erfindungsgemäße Emulsion Stearinsäuresalze als Emulgator, so ist es erfindungsgemäß bevorzugt, diesen Emulgator in einer Konzentration von 0,05 bis 0,5 Gewichts-%, bezogen auf das Gesamtgewicht der Emulsion einzusetzen.
Als Stearinsäuresalze werden dabei erfindungsgemäß bevorzugt Natrium- und Kaliumsalze eingesetzt.

Enthält die erfindungsgemäße Emulsion eine Kombination aus Natriumcetearylsulfat und Glycerylstearat SE als Emulgator, so ist es erfindungsgemäß bevorzugt, diese Stoffe in den folgenden Konzentrationen einzusetzen:
Es ist erfindungsgemäß vorteilhaft, wenn die erfindungsgemäße Emulsion Natriumcetearylsulfat in einer Menge von 0,1 bis 2 Gew.-%, bezogen auf das Gesamtgewicht der Emulsion, enthält.

Es ist erfindungsgemäß vorteilhaft, wenn die erfindungsgemäße Emulsion Glycerylmonostearat SE in einer Menge von 0,1 bis 5 Gew.-%, bezogen auf das Gesamtgewicht der Emulsion, enthält.

Erfindungsgemäß wird selbstemulgierendes Glycerylmonostearat mit der INCI Glyceryl Monostearate SE eingesetzt.

Erfindungsgemäß vorteilhaft beträgt das Gewichtsverhältnis in der Emulsion von Natriumcetearylsulfat zu Glycerylmonostearat von 1:1 bis 1:20.

Darüber hinaus kann die erfindungsgemäße Emulsion vorteilhaft Salze enthalten, insbesondere Meersalz.

Es ist erfindungsgemäß vorteilhaft, wenn die erfindungsgemäße Emulsion einen oder mehrere Wirkstoffe gewählt aus der Gruppe der Verbindungen Glycyrrhetinsäure, Harnstoff, Arctiin, alpha-Liponsäure, Folsäure, Phytoen, D-Biotin, Coenzym Q10, Hyaluronsäure, alpha-Glucosylrutin, Carnitin, Carnosin, Coffein, natürliche und/oder synthetische Isoflavonoide, Glycerylglucose, Kreatin, Kreatinin, Taurin, β-Alanin und/oder Licochalcon A, Panthenol, Tocopherol, Tocopherolacetat, Vitamin C, Vitamin C Derivate, Glycyrrhiza Inflata Root Extract, Magnolienextrakt enthält.

Außerdem sind erfindungsgemäß vorteilhafte Ausführungsformen der vorliegenden Erfindung dadurch gekennzeichnet, dass die Zubereitung Propylenglycol, Butylenglycol, 2-Methylpropan-1,3-diol, Ethylhexylglycerin, 1,2-Pentandiol, 1,2-Hexandiol, 1,2-Octandiol und/oder 1,2-Decandiol enthält. Dabei ist der Gehalt an 2-Methylpropan-1,3-diol, 1,2-Pentandiol und/oder 1,2-Hexandiol erfindungsgemäß bevorzugt.

Erfindungsgemäß vorteilhafte Ausführungsformen der vorliegenden Erfindung sind dadurch gekennzeichnet, dass die Emulsion einen oder mehrere UV-Filter enthält, gewählt aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 4-(tert.-Butyl)-4'-methoxydibenzoylmethan; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; 4-(tert.-Butyl)-4'-methoxydibenzoylmethan; 2-(4'-Diethylamino-2'-hydoxybenzoyl)-benzoesäurehexylester; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titandioxid; Zinkoxid.

Es ist erfindungsgemäß besonders bevorzugt, wenn die Emulsion frei ist von Propyl- und Butylparaben, 3-lod-2-propinylbutylcarbamat, 3-(4-Methylbenzyliden)-campher, 2-Hydroxy-4-methoxybenzophenon (Oxybenzon) und halogenhaltigen Verbindungen.

Die erfindungsgemäßen kosmetischen Emulsionen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben und/oder Aerosile® (CAS-Nr. 7631-86-9) und /oder Talkum, Polymethylsilsesquioxane, Nylon, Silica Dimethyl Silyate

Erfindungsgemäß bevorzugt ist es, wenn der kosmetische Schaum dadurch gekennzeichnet ist, dass die Emulsion mit Polymethylsilsequioxan modifizierte Tapiokastärke (INCI Tapioca starch + Polymethylsilsesquioxane) enthält. Diese wird erfindungsgemäß vorteilhaft in einer Konzentration von 0,1 bis 3 Gew.-%, bezogen auf das Gesamtgewicht der Emulsion eingesetzt.

Der erfindungsgemäße Schaum bzw. die erfindungsgemäße Emulsion haben erfindungsgemäß vorteilhafte einen pH-Wert von 5 bis 7,5.

Erfindungsgemäß vorteilhaft wird der erfindungsgemäße Schaum in einer Aerosoldose mit Entnahmeventil aufbewahrt und aus dieser heraus angewendet. Zur Anwendung wird die erfindungsgemäße Mischung aus Emulsion und Gas(en) durch Schütteln in der Aerosoldose zuerst durchmischt und anschließend über das Entnahmeventil entnommen und auf die Haut appliziert. Dabei zeigt sich ein weiterer erfindungsgemäßer Vorteil, der darin liegt, dass der erfindungsgemäße Schaum sich im Vergleich zu herkömmlichen Schäumen vollständiger aus der Aerosoldose entnehmen lässt, d.h. dass die so genannte "Resteentleerung" überraschend höher ist als bei vergleichbaren, herkömmlichen Schäumen. Darüber hinaus zeigt der erfindungsgemäße Schaum auch bei niedrigen pH-Werten eine überraschend gute Packmittel-Kompatibilität, d.h. dass beispielsweise Korrosionseffekte deutlich seltener und schwächer ausfallen als bei vergleichbaren, herkömmlichen Systemen.

Als Aerosoldosen mit Entnahmeventil können die üblichen für kosmetische Zwecke bekannten Aerosoldosen-Systeme eingesetzt werden.

Erfindungsgemäß ist daher auch eine Aerosoldose mit Entnahmeventil enthaltend den erfindungsgemäßen Schaum, sowie ein Verfahren zur Anwendung kosmetischer Schäume auf der Haut, welches dadurch gekennzeichnet ist, dass die erfindungsgemäße Mischung aus Emulsion und Gas(en) durch Schütteln in der Aerosoldose zuerst durchmischt und anschließend über das Entnahmeventil entnommen und auf die Haut appliziert wird.

Nicht zuletzt ist es erfindungsgemäß bevorzugt, wenn der erfindungsgemäße Schaum dadurch gekennzeichnet ist, dass er in einer Aerosoldose mit Entnahmeventil enthalten ist.

### Vergleichsversuch

| **Zusammensetzung** | Muster A | Muster B |
|---|---|---|
| Capryhc/Caphc Triglycerid | 3 | 3 |
| C15-19 Alkan | 4 | 4 |
| Isopropyl Palmitat | 3 | 3 |
| Shea Butter | 1 | 1 |
| Dimethicon | 1 | 1 |
| Glyceryl Stearate SE | 1,5 | |
| Natrium Cetearyl Sulfat | 0,15 | |
| PEG-40 Stearat | | 1,65 |
| Glycerin | 9,3 | 9,3 |
| 45% ige Natronlauge | 0,05 | 0,05 |
| Phenoxyethanol | 0,5 | 0,5 |
| Behenyl Alkohol | 2 | 2 |
| Carbomer | 0,1 | 0,1 |
| Wasser | 74,4 | 74,4 |
| | | |

| **Konservierungsbelastungstest** | | |
|---|---|---|
| | Keimzahl nach 14 d KBE | |
| Staph.areus, Staph.epidermis Beimpfung 170000 KBE | 71000 | 200000 |
| Pseudomonas aeruginosa Pseudomonas putida Burkholderia cepacia Beimpfung : 290000 | 700 | 1900 |
| Escherichia coli Enetrobacter gergoviae Klebsiella pneumoniae Beimpfung: 410000 | 6900 | > 200000 |
| Candida albicans Candida parapsilosis Pichia guilliermondii Beimpfung: 320000 | 160000 | 160000 |
| Aspergillus brasiliensis Beimpfung: 240000 | 320000 | 350000 |
| | | |

| **Stabilität** | | |
|---|---|---|
| Emulsion 2 Monate bei 40°C gelagert | i.O. | deutliche Wasserabscheidung |
| Schaum | | |
| 96% Emulsion + 4% Propan/Butan/i-Butan Druckstufe 2,7 bar | Cremiger, pflegender feinblasiger Schaum | Wässriger, mittelblasiger instabiler Schaum |

### Vergleichsversuch II

| **Zusammensetzung** | Muster #40 | Muster #41 |
|---|---|---|
| | | |
| Piroctone Olamin | 0,06 | 0,06 |
| C15-19 Alkan | 4 | 4 |
| Shea Butter | 1 | 1 |
| Mineralöl | 3 | 3 |
| Dimethicon | 1 | 1 |
| Isopropyl Palmitat | 3 | 3 |
| Glyceryl Stearate SE | 1,5 | 1,5 |
| Natrium Cetearyl Sulfat | 0,15 | 0,15 |
| Parfum | 0,35 | 0,35 |
| Glycerin | 9,3 | 9,3 |
| 45%-iqe Natronlauge | 0,06 | 0,06 |
| Phenoxyethanol | 0,9 | 0,9 |
| Behenyl Alkohol | 2 | 2 |
| Carbomer | 0,1 | 0,1 |
| Wasser | 73,58 | 72,58 |
| Ethanol | | 1 |

| **Stabilität** | | |
|---|---|---|
| Emulsion 6 Monate bei 40°C gelagert | i.O. | i.O. |

| Schaum | | |
|---|---|---|
| 94% Emulsion + 6% Propan/Butan/i-Butan Druckstufe 3,0 bar | Instabiler, grobblasiger Schaum | Cremiger, stabiler, fein blasiger Schaum |

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

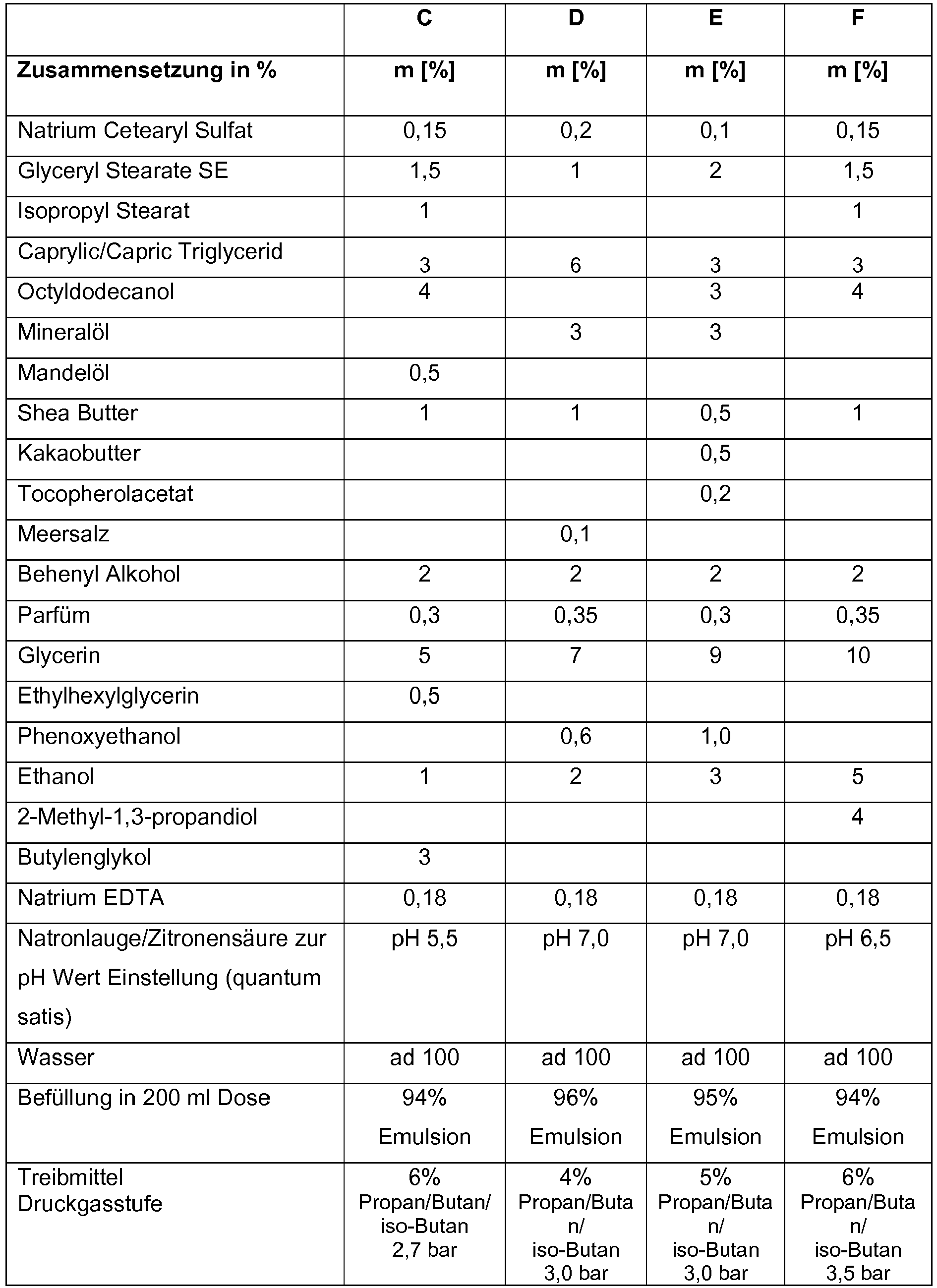

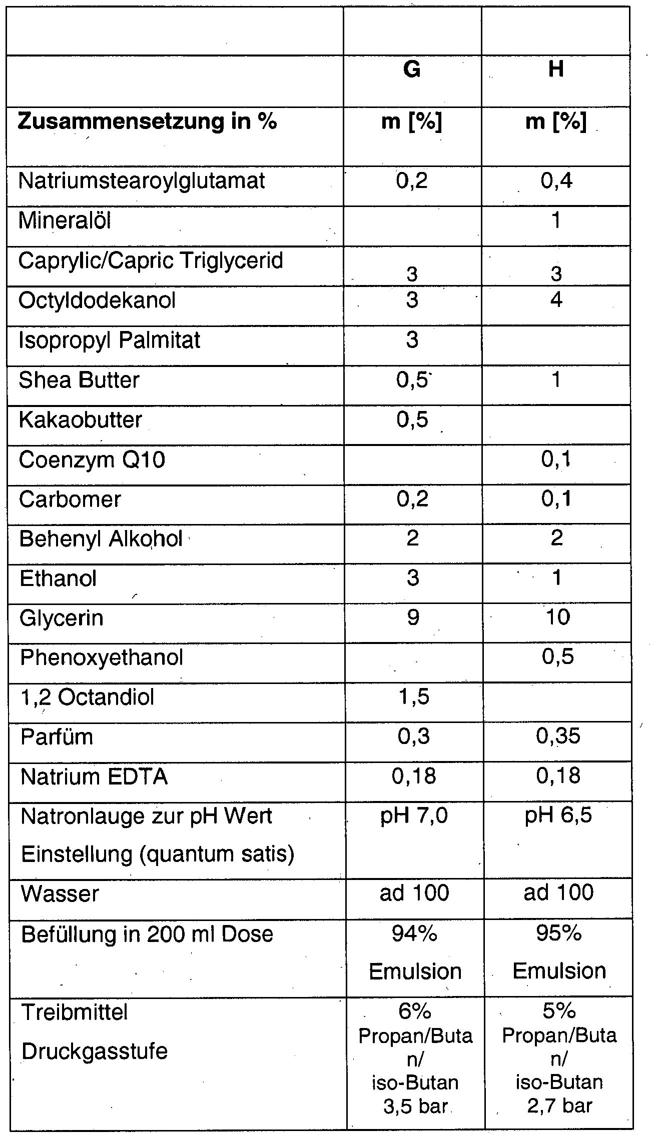

## Patentansprüche

1. Kosmetischer Schaum aus
a) einer O/W-Emulsion enthaltend Glycerin sowie
b) ein- und/oder mehrwertige Alkohole mit einer Kohlenstoffkettenlänge von weniger als 12 C-Atomen und
c) einem die Emulsion aufschäumenden Gas oder Gasgemisch aus Propan, n-Butan und/oder Isobutan,
wobei die Emulsion frei ist von Verbindungen mit Alkohol- oder Säurefunktion, die mit Polyethylenglycolen ganz oder teilweise verethert oder verestert sind (PEG-Derivaten) und die Emulsion einen oder mehrere Emulgatoren gewählt aus der Gruppe der Verbindungen Polyglycerinester , Polyglyceryl-3 Methylglucosedistearat, Cetearyl Glycosid, Natriumstearoylglutamat, Cetylphosphat, Stearinsäuresalze oder eine Kombination aus Natriumcetearylsulfat und Glycerylstearat SE enthält, **dadurch gekennzeichnet, dass** die Lipidphase der Emulsion Stearylalkohol bzw. Behenylalkohol enthält.

2. Kosmetischer Schaum nach Anspruch 1, **dadurch gekennzeichnet, dass** die Emulsion von 3 bis 20 Gewichts-%, bezogen auf das Gesamtgewicht der Emulsion Glycerin enthält.

3. Kosmetischer Schaum nach einem der vorhergehenden Ansprüche, **dadurch gekennzeichnet, dass** die Gesamtkonzentration an ein- und mehrwertigen Alkoholen mit einer Kohlenstoffkettenlänge von weniger als 12 C-Atomen in der Emulsion 0,5 bis 10 Gewichts-%, bezogen auf das Gesamtgewicht der Emulsion, beträgt.

4. Kosmetischer Schaum nach einem der vorhergehenden Ansprüche, **dadurch gekennzeichnet, dass** die ein- und/oder mehrwertigen Alkohole mit einer Kohlenstoffkettenlänge von weniger als 12 C-Atomen ausgewählt werden aus der Gruppe der Verbindungen Ethanol, Phenoxyethanol, 1,2-Pentandiol, 1,2-Hexandiol, 1,2-Octandiol, 2-Methyl-1,3-propandiol, 1,2-Decandiol, Ethylhexylglycerin, Benzylalkohol, Phenethylalkohol.

5. Kosmetischer Schaum nach einem der vorhergehenden Ansprüche, **dadurch gekennzeichnet, dass** der Schaum gebildet wird aus 90-96 Gew.-% Emulsion und 4 bis 10 Gew.-% Gas bzw. Gasgemisch.

6. Kosmetischer Schaum nach einem der vorhergehenden Ansprüche, **dadurch gekennzeichnet, dass** die Emulsion frei ist von Parabenen, Isothiazolinonen und 3-Iodpropargyl-*N*-butylcarbamat (IPBC) sowie halogenhaltigen Verbindungen.

7. Kosmetischer Schaum nach einem der vorhergehenden Ansprüche, **dadurch gekennzeichnet, dass** die Lipidphase der Emulsion Mandelöl, Kakaobutter und/oder Sheabutter enthält.

8. Kosmetischer Schaum nach einem der vorhergehenden Ansprüche, **dadurch gekennzeichnet, dass** der Gewichtsanteil der Lipidphase an der Emulsion 7 bis 25 Gew.-%, bezogen auf das Gesamtgewicht der Emulsion beträgt.

9. Kosmetischer Schaum nach einem der vorhergehenden Ansprüche, **dadurch gekennzeichnet, dass** die Emulsion Emulgatoren in einer Gesamtkonzentration von 0,1 bis 8 Gewichts-%, bezogen auf das Gesamtgewicht der Emulsion, enthält.

10. Aerosoldose mit Entnahmeventil enthaltend einen kosmetischen Schaum nach einem der Ansprüche 1 bis 9.

## Claims

1. Cosmetic foam from
a) an O/W emulsion comprising glycerin and
b) monohydric and/or polyhydric alcohols having a carbon chain length of less than 12 carbon atoms and
c) a gas or gas mixture composed of propane, n-butane and/or isobutane foaming the emulsion,
wherein the emulsion is free of compounds having alcohol or acid functions which have been wholly or partially etherified or esterified with polyethylene glycols (PEG derivatives) and the emulsion comprises one or more emulsifiers selected from the group of compounds comprising polyglycerin esters, polyglyceryl-3 methylglucose distearate, cetearyl glycoside, sodium stearoyl glutamate, cetyl phosphate, stearic acid salts or a combination of sodium cetearyl sulfate and glyceryl stearate SE, **characterized in that** the lipid phase of the emulsion comprises stearyl alcohol or behenyl alcohol.

2. Cosmetic foam according to Claim 1, **characterized in that** the emulsion comprises from 3 to 20% by weight glycerin, based on the total weight of the emulsion.

3. Cosmetic foam according to either of the preceding claims, **characterized in that** the total concentration of monohydric and polyhydric alcohols having a chain length of less than 12 carbon atoms in the emulsion is 0.5 to 10% by weight, based on the total weight of the emulsion.

4. Cosmetic foam according to any of the preceding claims, **characterized in that** the monohydric and/or polyhydric alcohols having a carbon chain length of less than 12 carbon atoms are selected from the group of compounds comprising ethanol, phenoxyethanol, 1,2-pentanediol, 1,2-hexanediol, 1,2-octanediol, 2-methyl-1,3-propanediol, 1,2-decanediol, ethylhexylglycerin, benzyl alcohol, phenylethyl alcohol.

5. Cosmetic foam according to any of the preceding claims, **characterized in that** the foam is formed from 90-96% by weight emulsion and 4 to 10% by weight gas or gas mixture.

6. Cosmetic foam according to any of the preceding claims, **characterized in that** the emulsion is free of parabens, isothiazolinones and 3-iodopropargyl N-butylcarbamate (IPBC) and also halogen-containing compounds.

7. Cosmetic foam according to any of the preceding claims, **characterized in that** the lipid phase of the emulsion comprises almond oil, cocoa butter and/or shea butter.

8. Cosmetic foam according to any of the preceding claims, **characterized in that** the proportion by weight of the lipid phase in the emulsion is 7 to 25% by weight, based on the total weight of the emulsion.

9. Cosmetic foam according to any of the preceding claims, **characterized in that** the emulsion comprises emulsifiers at a total concentration of 0.1 to 8% by weight, based on the total weight of the emulsion.

10. Aerosol can with release valve comprising a cosmetic foam according to any of Claims 1 to 9.

## Revendications

1. Mousse cosmétique composée
a) d'une émulsion huile/eau contenant de la glycérine ainsi que
b) d'alcools monovalents et/ou polyvalents dotés d'une longueur de chaîne carbonée inférieure à 12 atomes de C et
c) d'un gaz ou d'un mélange de gaz moussant l'émulsion, composé de propane, de n-butane et/ou d'isobutane,
l'émulsion étant exempte de composés dotés d'une fonction alcool ou acide, qui sont totalement ou partiellement éthérés ou estérifiés avec des polyéthylèneglycols (dérivés de PEG) et l'émulsion contenant un ou plusieurs émulsifiants choisis dans le groupe des composés comprenant des esters de polyglycérine, un polyglycéryl-3-méthylglucosedistéarate, un glycoside de cétéaryle, le stéaroylglutamate de sodium, le phosphate de cétyle, des sels d'acide stéarique ou une combinaison de cétéarylsulfate de sodium et de stéarate de glycéryle SE, **caractérisée en ce que** la phase lipidique de l'émulsion contient de l'alcool stéarylique ou de l'alcool béhénylique.

2. Mousse cosmétique selon la revendication 1, **caractérisée en ce que** l'émulsion contient de 3 à 20 % en poids de glycérine, par rapport au poids total de l'émulsion.

3. Mousse cosmétique selon l'une quelconque des revendications précédentes, **caractérisée en ce que** la concentration totale en alcools monovalents et/ou polyvalents dotés d'une longueur de chaîne carbonée inférieure à 12 atomes de C dans l'émulsion est de 0,5 à 10 % en poids, par rapport au poids total de l'émulsion.

4. Mousse cosmétique selon l'une quelconque des revendications précédentes, **caractérisée en ce que** les alcools monovalents et/ou polyvalents dotés d'une longueur de chaîne carbonée inférieure à 12 atomes de C sont choisis dans le groupe des composés comprenant l'éthanol, le phénoxyéthanol, le 1,2-pentanediol, le 1,2-hexanediol, le 1,2-octanediol, le 2-méthyl-1,3-propanediol, le 1,2-décanediol, l'éthylhexylglycérine, l'alcool benzylique, l'alcool phénéthylique.

5. Mousse cosmétique selon l'une quelconque des revendications précédentes, **caractérisée en ce que** la mousse est formée à partir de 90 à 96 % en poids d'émulsion et de 4 à 10 % en poids de gaz ou de mélange de gaz.

6. Mousse cosmétique selon l'une quelconque des revendications précédentes, **caractérisée en ce que** l'émulsion est exempte de parabènes, d'isothiazolinones et de 3-iodopropargyl-N-butylcarbamate (IPBC) ainsi que de composés contenant un halogène.

7. Mousse cosmétique selon l'une quelconque des revendications précédentes, **caractérisée en ce que** la phase lipidique de l'émulsion contient de l'huile d'amande, du beurre de cacao et/ou du beurre de karité.

8. Mousse cosmétique selon l'une quelconque des revendications précédentes, **caractérisée en ce que** la proportion en poids de la phase lipidique dans l'émulsion est de 7 à 25 % en poids, par rapport au poids total de l'émulsion.

9. Mousse cosmétique selon l'une quelconque des revendications précédentes, **caractérisée en ce que** l'émulsion contient des émulsifiants en une concentration totale de 0,1 à 8 % en poids, par rapport au poids total de l'émulsion.

10. Récipient d'aérosol doté d'une soupape de soutirage contenant une mousse cosmétique selon l'une quelconque des revendications 1 à 9.
